# EUROPÄISCHE PATENTANMELDUNG

(11) **EP 0 641 777 A1**
(43) Veröffentlichungstag der Anmeldung: **08.03.1995**
(21) Anmeldenummer: 94113464.5
(22) Anmeldetag: 29.08.1994
(51) Int. Cl.: C07C 407/00, C07C 409/26

(54) **Verfahren und Vorrichtung zur kontinuierlichen Herstellung von Peroxycarbonsäuren**

(30) Priorität: 08.09.1993 DE 4330465
(71) Anmelder: LAPORTE GmbH, D-82049 Höllriegelskreuth (DE)
(72) Erfinder: Stoll, Jürgen Dipl.-Ing., D-68167 Mannheim (DE); Schlenker, Dr. Walter, D-67117 Limburgerhof (DE); Schober, Dr. Klaus, D-67259 Heuchelheim (DE)
(74) Vertreter: Huber, Bernhard, Dipl.-Chem.

(57) **Zusammenfassung**

Es wird ein Verfahren zur kontinuierlichen Herstellung von Peroxycarbonsäuren durch Umsetzung einer Carbonsäure mit Wasserstoffperoxid in einem wässerigen Medium in Gegenwart eines Katalysators bei einer Reaktionstemperatur im Bereich von 40 bis 60°C, sowie eine Vorrichtung zur Durchführung dieses Verfahrens beschrieben.

Die Vorrichtung umfaßt einen Verweilreaktor 1 zur Aufnahme des Reaktionsmediums, die mit einer Heizung 10 versehen ist, zwei mit Rückschlagventilen 9,9 versehene Zuleitungen 2,2, die mit je einer Dosierpumpe 3,3 zur Zuführung der Reaktionskomponenten aus den Vorratsbehältern 11, 12 in die Zuleitungen 2,2 versehen sind.

## Beschreibung

Die Erfindung betrifft ein Verfahren zur kontinuierlichen und kontrollierten Herstellung von Peroxycarbonsäuren, insbesondere von Peroxyessigsäuren, sowie eine Vorrichtung zur Durchführung dieses Verfahrens.

Peroxycarbonsäuren werden im Laboratorium und im technischen Maßstab zur Gewinnung von Epoxiden nach der sogenannten Prilezhaev-Reaktion (vgl. Trahaowsky, Oxidation in Organic Chemistry, Tl.C, Seite 211 bis 252, New York, Academic Press 1978) und zur Hydroxylierung, zur Baeyer-Villiger-Oxidation und zu speziellen Oxidationsprozessen verwendet. Darüber hinaus werden Peroxycarbonsäuren in zunehmendem Maße in der chemischen und verwandten Industrie, z.B. in der Nahrungsmittelindustrie, pharmazeutischen Industrie und Papierindustrie, oder zur Wasserbehandlung als Desinfektionsmittel eingesetzt.

In diesen Industriezweigen besteht ein ständig steigender Bedarf an Desinfektionsmitteln zur Entkeimung von Produktionsanlagen. Hierbei kommen üblicherweise Desinfektionsmittel, wie z.B. Natriumhypochlorit, Chlordioxid, Halogencarbonsäuren, Aldehyde, quaternäre Ammoniumverbindungen, Wasserstoffperoxid und Peroxycarbonsäure, insbesondere Peroxyessigsäure (Peressigsäure) zum Einsatz.

Insbesondere Wasserstoffperoxid und die Peroxyessigsäuren können dabei als besonders umweltfreundlich angesehen werden, da sie während und nach dem Einsatz zu ökologisch unbedenklichen Abbauprodukten wie Wasser bzw. Essigsäure abgebaut werden. Aus diesem Grund kommt der Anwendung von Peroxycarbonsäure, insbesondere von Peroxyessigsäure, gerade in der Nahrungsmittelindustrie eine ständig wachsende Bedeutung zu.

Peroxycarbonsäuren, wie z.B. Peroxyessigsäure, sind als organische Peroxiyde thermisch nicht stabil und können bei einer übermäßigen Erwärmung oder durch vorhandene Verunreinigungen heftigen Zersetzungsreaktionen unterliegen, woraus sich große Probleme beim Transport und bei der Lagerung ergeben. Peroxyessigsäure explodiert z.B. in > 50 Vol.-% organischen und > 70 Vol.-% wäßrigen Lösungen, so daß man sie vielfach in situ, d.h. im Reaktionsgefäß, z.B. aus Essigsäure und H2O2 in Gegenwart von Säuren herstellt.

Der Umgang mit niedermolekularen Peroxycarbonsäuren, wie z.B. Peroxyessigsäure, ist für den Anwender jedoch auch deshalb problematisch, da diese Stoffe einen stechenden Geruch aufweisen und die Dämpfe auf Augen, Haut und Schleimhäute stark ätzend wirken.

Bei den im Handel befindlichen, üblicherweise zu Desinfektionszwecken eingesetzten Peroxycarbonsäuren, insbesondere Peroxyessigsäure, handelt es sich um sogenannte Gleichgewichtsperoxycarbonsäuren. Die Konzentrationen an z.B. Peroxyessigsäure in diesen Desinfektionsmitteln liegen meistens zwischen 5 und 15 Gew.-%. Das Konzentrat enthält die Komponenten Wasserstoffperoxid, Essigsäure, Peroxyessigsäure, Wasser, einen sauren Katalysator und Stabilisatoren. Davon stehen die Komponenten Wasserstoffperoxid, Essigsäure, Peroxyessigsäure und Wasser gemäß der nachfolgenden Gleichung in einem chemischen Gleichgewicht: 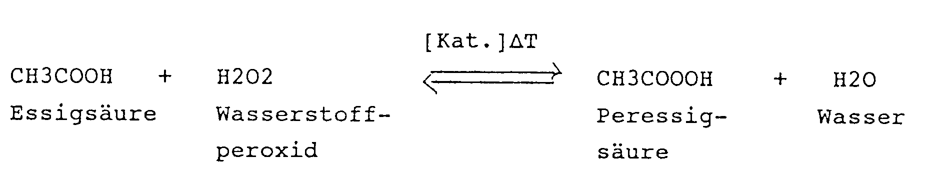
Der Gehalt an Peressigsäure im Gleichgewicht ist deshalb von der Konzentration der Reaktionspartner Essigsäure und Wasserstoffperoxid abhängig, sowie von der Temperatur, und die Einstellung des Gleichgewichts ist säurekatalysiert.

Aufgabestellung der vorliegenden Erfindung ist es, die vorstehend im Zusammenhang mit der Herstellung, Behandlung und Anwendung von Peroxycarbonsäuren verbundenen Nachteile und Probleme, insbesondere den stechenden Geruch und die Reiz- und Verätzungsgefahr von Augen, Haut und Schleimhäuten des Bedienungspersonals, zu vermeiden und ein Verfahren und eine Vorrichtung bereitzustellen, mit der auch eine rasche Einstellung des Reaktionsgleichgewichts und damit eine gute kontinuierliche Durchführung des Verfahrens erreicht werden kann.

Dies wird erfindungsgemäß durch ein Verfahren und eine Vorrichtung zur Herstellung von Peroxycarbonsäuren, insbesondere Peroxyessigsäure, erzielt, bei der die Peroxycarbonsäure in einer geschlossenen Anlage und nur bei direkten Bedarf und in der entsprechenden gewünschten Menge erzeugt wird, wobei ein direkter Kontakt von Personen bei der Herstellung der Peroxycarbonsäuren und bei deren Anwendung, z.B. als Desinfektionsmittel, vermieden wird.

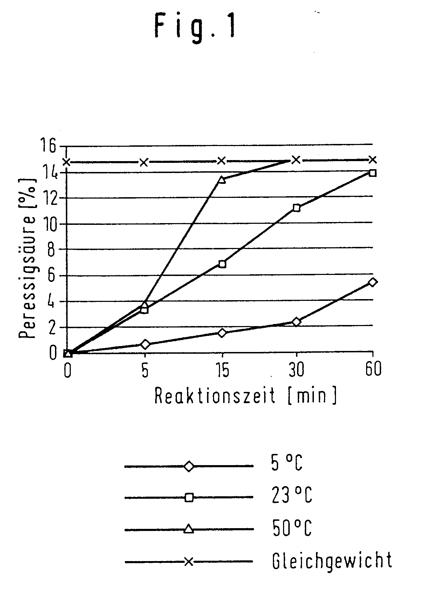

Die Anmelderin hat den Temperatureinfluß auf die Einstellung des Reaktionsgleichgewichts untersucht, und die erhaltenen Ergebnisse sind in der nachfolgenden Abbildung 1 dargestellt. Den Meßwerten liegt ein Gemisch aus 14,7 % Wasserstoffperoxid, 37,5 % Essigsäure, 0,5 % Stabilisator, 11,25 % Schwefelsäure und 36,05 % Wasser zugrunde.

Aus der Figur 1 folgt, daß durch Aufheizen der Reaktionsgemisches auf ca. 50°C das Reaktionsgleichgewicht, d.h. die optimale Konzentration an der gewünschten Peroxyessigsäure, bereits nach ca. 30 Minuten erreicht wird. Bei Raumtemperatur oder bei tieferen Temperaturen (z.B. bei 5°C in einem unbeheizten Raum im Winter) wird das Gleichgewicht hingegen wesentlich später erreicht, und zwar nach einer Zeitdauer, die für einen kontinuierlichen Prozeß, um z.B. den Bedarf an Peroxyessigsäure zur Desinfektion in modernen Reinigungsanlagen (z.B. Flaschenreinigungsanlagen in der Getränkeindustrie) oder in Produktionsanlagen ( z.B. Tankdesinfektion in Brauereien) decken zu können, zu lange ist.

Aus der EP-A-0269345 ist ein Verfahren und eine Vorrichtung zur Verdünnung eines Reaktionsprodukts, insbesondere einer Peroxysäure, zur Herstellung einer Desinfektionslösung bekannt, wonach man eine Umsetzung zwischen 2 oder mehr Reaktanden in einem Reaktor durchführt, das Reaktionsprodukt verdünnt, die Konzentration des Reaktionsprodukts nach der Verdünnung unter Verwendung eines elektrochemischen Sensors verfolgt, und die Verdünnung des Reaktionsprodukts gemäß der Anzeige dieses Sensors regelt. Im Reaktor ist jedoch keine Einrichtung zur Aufheizung des Reaktionsgemisches und damit zur Durchführung des Verfahrens unter Erhöhung der Temperatur zur rascheren Gleichgewichtseinstellung vorhanden. Mit diesem Reaktor konnten deshalb die aktuellen Anforderungen, wie sie im Zusammenhang mit der Herstellung von Peroxyessigsäuren, z.B. in Großbetrieben der Nahrungsmittelindustrie, gestellt werden, nicht zufriedenstellend, erfüllt werden.

Gegenstand der Erfindung ist ein Verfahren zur kontinuierlichen Herstellung von Peroxycarbonsäuren durch Umsetzung einer Carbonsäure mit Wasserstoffperoxid in einem wäßrigen Medium in Gegenwart eines Katalysators, dadurch gekennzeichnet, daß man die Umsetzung bei einer Temperatur im Bereich von 40 bis 60°C durchführt.

Zweckmäßige Ausgestaltungen dieses Verfahrens sind Gegenstand der Ansprüche 2 bis 13.

In einer zweckmäßigen Ausgestaltung des erfindungsgemäßen Verfahrens wird das Wasserstoffperoxid und das Carbonsäure/Katalysator-Gemisch einem Verweilreaktor zur Aufnahme des Reaktionsgemisches in 2 getrennten Zuleitungen kontinuierlich zugeführt und die gebildete Peroxycarbonsäure nach Einstellung des Reaktionsgleichgewichts kontinuierlich vom Verweilreaktor abgezogen.

Als Wasserstoffperoxid verwendet man im erfindungsgemäßen Verfahren vorzugsweise ein solches mit einer Konzentration von 30 bis 50 Gewichtsprozent.

Als Katalysatoren können alle für die Peroxycarbonsäureherstellung durch Umsetzung einer Carbonsäure mit Wasserstoffperoxid bekannten und üblichen Katalysatoren, eingesetzt werden. Erfindungsgemäß eingesetzte Katalysatoren sind vorzugsweise Mineralsäuren, wie z.B. Phosphorsäure oder Salpetersäure, und insbesondere Schwefelsäure. Salzsäure ist als Mineralsäure jedoch nicht geeignet, weil sie leicht zu Zersetzungsreaktionen führen kann. Im Prinzip können jedoch jegliche organischen (z.B. Phosphonsäuren) oder anorganischen Säuren als Katalysatoren eingesetzt werden, vorausgesetzt, sie sind gegen das oxidierend wirkende Medium stabil und fördern nicht die Zersetzung der Aktivsauerstoffträger.

Die Umsetzung wird bei einer Temperatur im Bereich von 40 bis 60°C durchgeführt, bevorzugt bei einer Temperatur von ca. 50 bis 60°C.

Die Konzentration der Peroxycarbonsäure im Reaktionsmedium kann in einem breiten Bereich variieren; vorzugsweise beträgt die Konzentration 10 bis 30 Gew.-%, insbesondere 10 bis 25 Gew.-%, und in erster Linie 13 bis 15 Gew.-%.

Die Reaktionstemperatur liegt im allgemeinen im Bereich von 40 bis 60°C, insbesondere im Bereich von 50 bis 55°C, und in erster Linie wird die Umsetzung bei einer Temperatur von ca. 50°C durchgeführt.

Wird bei höheren Temperaturen als 60°C gearbeitet, so wird die Zersetzung der Peroxycarbonsäuren in unerwünschtem Ausmaß beschleunigt; wird bei einer Temperatur von < 40°C gearbeitet, so wird das Reaktionsgleichgewicht erst nach einer für eine kontinuierliche Durchführung des Verfahrens ungeeignet langen Zeit erreicht.

Gegenstand der vorliegenden Erfindung ist auch eine Vorrichtung, die insbesondere zur kontinuierlichen Herstellung von Peroxycarbonsäuren nach dem erfindungsgemäßen Verfahren geeignet ist, umfassend: einen Verweilreaktor zur Aufnahme des Reaktionsmediums, der mit einer Heizeinrichtung versehen ist, und 2 mit Rückschlagventilen versehene Zuleitungen, die mit je einer Dosierpumpe zur Zuführung der Reaktionskomponenten aus dem Vorratsbehälter in die Zuleitungen versehen sind.

Zweckmäßige Ausgestaltungen der erfindungsgemäßen Vorrichtung sind Gegenstand der Ansprüche 15 bis 26.

Als Carbonsäure wird vorzugsweise eine C₁-C₆-Carbonsäure oder Oligocarbonsäure eingesetzt, und insbesondere Ameisensäure oder Essigsäure.

In einer bevorzugten Ausführungsform des erfindungsgemäßen Verfahrens wird die vom Verweilreaktor abgezogene Peroxycarbonsäure, z.B. Peroxyessigsäure, direkt dem Verwendungszweck zugeführt, z.B. einer zu desinfizierenden Leitung, oder Flaschenreinigungsanlage in der Getränkeindustrie, oder Produktionsanlagen, z.B. Gär-, Lager-und Drucktanks in Brauereien, oder einem anderen Desinfektionsmittelkreislauf. Auf diese Weise läßt sich durch Steuerung des Verfahrens (Steuerung der Menge an erzeugtem Peroxycarbonsäure-Konzentrat) und Verwendung eines geschlossenen Kreislaufs bis zur beabsichtigten Verwendung, z.B. bis zu den zu desinfizierenden Anlagen, ein Kontakt der gesundheitsschädlichen Peroxyessigsäuren oder deren Dämpfen und die damit verbundenen Beeinträchtigungen, wie Reizungen und Verätzungen von Augen, Schleimhaut und Haut, vermeiden.

Durch die erfindungsgemäße Reaktionstemperatur ist es außerdem möglich, das Reaktionsgleichgewicht in einem für eine kontinuierliche und leicht steuerbare Durchführung des Verfahrens ausreichend kurzen Zeit, z.B. in ca. 30 Minuten, zu erreichen, wodurch eine leichte Steuerbarkeit des Verfahrens und damit eine Anpassung der nach dem Verfahren hergestellten Menge an den jeweiligen Bedarf möglich ist, und damit eine mit einer Zwischenlagerung verbundene weitere Behandlung der gesundheitsschädlichen Peroxycarbonsäuren vermieden werden kann.

Das Volumen des Verweilreaktors wird in erster Linie abhängig von der gefordeten Leistung (d.h. dem Ausstoß an Peroxycarbonsäurekonzentrat/Stunde) bestimmt. Die Aufheizung des Reaktionsgemisches kann durch ein Innenheizung, z.B. in Form einer elektrischen Heizschlange, oder aber auch durch eine Außenheizung, z.B. in Form eines den Reaktor umgebenden Hohlmantels, durch den eine Heizflüssigkeit oder Dampf strömt, erfolgen. Wenn der Reaktor aus einem Material mit einer schlechten Wärmeleitfähigkeit besteht, z.B. aus einem gegenüber den Komponenten des Reaktionsgemisches beständige Material, wie z.B. Kunststoff, so erfolgt die Beheizung vorzugsweise über einen Innenheizung.

Die Zuführung der Reaktionskomponenten aus den Vorratsbehältern in die Zuleitungen zum Verweilreaktor erfolgt über Dosierpumpen mit Durchflußkontrolle. Die Sauglanzen in den Vorratsbehältern sind mit Leermeldeeinrichtungen ausgestattet.

Die beiden Vorratsbehälter enthalten die Komponenten für die Herstellung der Peroxycarbonsäuren auf solche Weise, daß das Wasserstoffperoxid von der Carbonsäurekomponente getrennt aufbewahrt wird.

Vorzugsweise weist die erfindungsgemäße Vorrichtung einen Drucksensor auf, der sich am Ausgang des Verweilreaktors befindet; zweckmäßiger ist dieser Drucksensor mit einer Einrichtung gekoppelt, die bei einer Überschreitung eines Druckgrenzwertes die Vorrichtung automatisch mit Wasser spült. Auf diese Weise kann eine hohe Betriebssicherheit gewährleistet werden, weil bei der Überschreitung eines Druckgrenzwertes, die z.B. durch eine unvorhergesehene Zersetzungreaktion ausgelöst werden kann, dann die Anlage über den Druck-Sensor und die damit gekoppelte Einrichtung automatisch mit Wasser gespült werden kann.

Zweckmäßigerweise ist die Vorrichtung auch mit einem mechanischen Überdruckventil ausgerüstet, durch das auch bei einer Unterbrechung der Stromversorgung ein Druckabbau im Reaktor erfolgen kann.

Für eine einwandfreie und einfache Wartung der Anlage ist es auch zweckmäßig, die Vorrichtung mit einem Entlüftungsventil und einem Ablaßventil zur Entlüftung bzw. Entleerung und zur Spülung mit Wasser zu versehen. Die Ableitung der durch die Wasserspülung über das Ablaßventil ausgedrückten Peroxycarbonsäure erfolgt dabei zweckmäßig über ein mit der Vorrichtung verbundenes Neutralisationsgefäß.

In einer besonders zweckmäßigen Ausführungsform ist die Vorrichtung mit geeigneten Einrichtungen zur direkten Dosierung der aus dem Verweilreaktor abgezogenen Peroxycarbonsäure zum Ort der Verwendung versehen. Auf diese Weise lassen sich durch eine geeignete Steuerung des Verfahrens, d.h. des hergestellten Peroxycarbonsäurekonzentrats/Stunde und direkte Zuführung zum Ort der Verwendung (z.B. zu den mit Peroxycarbonsäure zu desinfezierenden Flaschenreinigungsanlagen der Getränkeindustrie, Produktionsanlagen, z.B. Gär-, Lager- und Drucktanks von Brauereien usw.) eine Zwischenlagerung und ein direkter Kontakt der gesundheitsschädlichen Peroxycarbonsäuren mit dem Bedienungspersonal vermeiden. Üblicherweise wird deshalb das Peroxycarbonsäurekonzentrat z.B. direkt in die zu desinfezierenden Leitungen oder in einen Desinfektionsmittelkreislauf dosiert, wobei die Dosierung über die Steuerung des Verfahrens zur Herstellung der Peroxycarbonsäuren (Ausstoß an Peroxycarbonsäurekonzentrat Stunde) in Koppelung mit elektrochemischen Sensoren gesteuert werden kann.

In der erfindungsgemäßen Vorrichtung bestehen vorzugsweise mindestens die mit den Ausgangs- oder Endprodukten (Carbonsäure, Wasserstoffperoxid, Mineralsäurekatalysator, Peroxycarbonsäure) in Berührung kommenden Teile der Vorrichtung aus einem gegenüber diesen Substanzen korrosionsbeständigen Material. Solche Materialien sind z.B. Kunststoffe, insbesondere Polyvinylidenfluorid (PVDF) und/oder Polytetrafluorethylen (PDFE), Glas, oder auch ein gegebenenfalls zusätzlich mit einem korrosionsbeständigen Überzug, wie z.B. Kunststoff oder Glas, beschichteter korrosionsbeständiger Stahl und/oder NE-Metall.

Der Verweilreaktor ist, insbesondere wenn er mit einer Innenheizung versehen ist, mit einem wärmeisolierenden Mantel, z.B. einem gegebenenfalls mit Wärmeisolationsmaterial gefüllten Hohlmantel, oder einer Beschichtung aus einem wärmeisolierenden Material versehen. Die Heizung des Verweilreaktors kann zweckmäßigerweise stufenlos geregelt werden. Als Verweilreaktor kann im wesentlichen jeder dafür bekannte und geeignete übliche Wärmeaustauscher oder Verweilreaktor eingesetzt werden, wobei der Verweilreaktor auch in eine temperaturgeregelte Heizstrecke und in eine thermisch isolierte Verweilstrecke unterteilt sein kann.

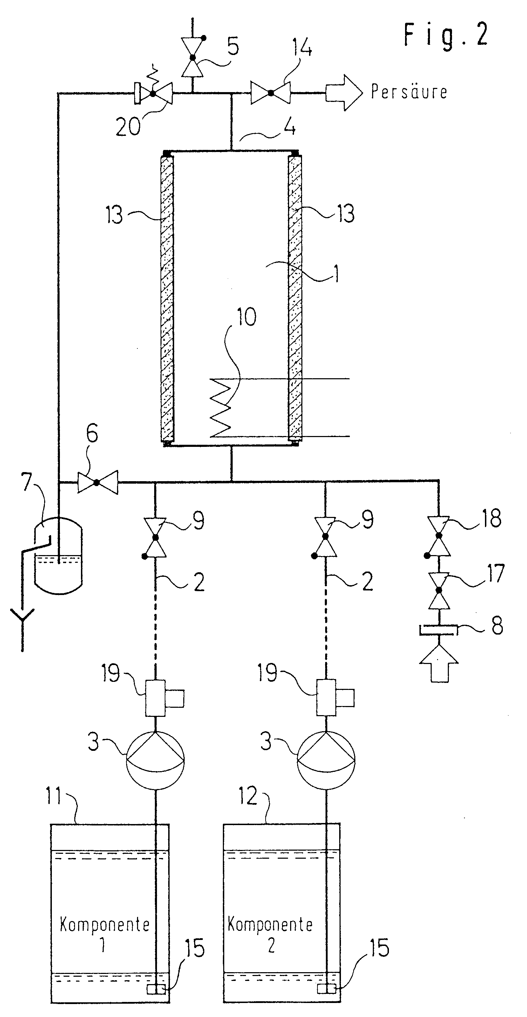

Die Figur 2 ist eine schematische Darstellung einer erfindungsgemäßen Vorrichtung.

Die vorliegende Erfindung wird nun unter Bezugnahme auf die durch Figur 2 dargestellte Vorrichtung näher erleutert, ohne ihren Rahmen darauf zu beschränken.

Die in Figur 2 dargestellte Ausführungsform einer Vorrichtung zur Durchführung des erfindungsgemäßen Verfahrens zur kontinuierlichen Herstellung von Peroxycarbonsäuren durch Umsetzung einer Carbonsäure mit Wasserstoffperoxid in Gegenwart eines Katalysators umfaßt einen Verweilreaktor 1 zur Aufnahme des Reaktionsmediums. Der Verweilreaktor ist mit einer elektrischen Innenheizung 10, die stufenlos regelbar ist, versehen. Der Verweilreaktor ist mit 2 mit Rückschlagventilen 9,9 versehenen Zuleitungen 2,2, die mit je einer Dosierpumpe 3,3 zur Zuführung der Reaktionskomponenten (Komponente 1 und Komponente 2) aus den Vorratsbehältern 11, 12 in die Zuleitungen 2,2 versehen sind, verbunden. Der Verweilreaktor ist mit einer Wärmeisolationsummantelung 13 versehen. Am Ausgang des Reaktors befindet sich ein Drucksensor 4 (nicht dargestellt) als Überdruckkontrolle, der mit dem Magnetventil 17 gekoppelt ist, welches sich bei einer Überschreitung eines Druckgrenzwertes öffnet, wodurch die Vorrichtung automatisch mit Wasser gespült wird. Die Vorrichtung weist außerdem ein mechanisches Überdruckventil 20 auf, das selbst bei einer Unterbrechung der Stromversorgung (z.B. Ausfall der öffentlichen Stromversorgung) einen Druckabbau im Reaktor bewirken kann. Die Vorrichtung weist außerdem ein Entlüftungsventil 5 und ein Ablaßventil 6 zur Entlüftung bzw. Entleerung auf. Sie ist außerdem mit einem Neutralisationsgefäß 7 versehen, über das die Ableitung der durch die Wasserspülung über das Überdruckventil 20 ausgedrückten bzw. über das Ablaßventil 6 abgelassenen Peroxycarbonsäure erfolgt; das Neutralisationsgefäß ist als Überlaufgefäß ausgebildet. Die Dosierung der Peroxycarbonsäure erfolgt auf Anforderung des Verbrauchers durch Ansteuerung des Magnetventils 14 und der Dosierpumpen 3,3, welche über Sauglanzen mit Füllstandssensoren 15,15 die Komponenten aus den Behältern 11,12 ansaugen. Die Vorratsbehälter 11 und 12 sind in einer beispielhaften Ausführungsform als Kontainer mit 800 l Inhalt, und in einer anderen beispielhaften Ausführungsform als Faß mit 200 l Inhalt ausgebildet. Dem Wasseranschluß 8 sind ein Magnetventil 17 und ein Kugelrückschlagventil 18 nachgeschaltet. Zwischen den Dosierpumpen 3,3 und den Rückschlagventilen 9,9 befinden sich Einrichtungen 19,19 zur Durchflußüberwachung.

Mit der in Figur 2 dargestellten Vorrichtung wurde das erfindungsgemäße Verfahren mit einer Reaktorleistung von 400 g Peroxyessigsäure/Stunde bzw. 2,76 kg eines durch den Reaktor innerhalb einer Stunde kontinuierlich erzeugten 15 %-igen Peroxyessigsäurekonzentrats durchgeführt. Die Temperatur der Heizstrecke betrug ca. 50°C, wodurch sich in ca. 30 Minuten ein Gleichgewichtsgemisch mit einer ca. 15 %-igen Peroxyessigsäure erhalten ließ. Das Reaktorvolumen betrug nur 1,5 l, was für die angegebene Reaktorleistung von 400 g Peroxyessigsäure/Stunde ausreichend war.

Durch eine weitere Temperaturerhöhung und/oder durch eine Aufkonzentrierung der Reaktionspartner läßt sich eine weitere Beschleunigung der Reaktion (kürzere Zeit bis zu Erreichung des Gleichgewichtsreaktionsgemisches) und gegebenenfalls eine weitere Verringerung des Reaktorvolumens erreichen.

Mit dem erfindungsgemäßen Verfahren und der erfindungsgemäßen Vorrichtung ist es deshalb möglich, je nach Auswahl und Konzentration der Komponenten und der Wahl der Reaktionstemperatur wäßrige Konzentrate von Peroxycarbonsäuren mit unterschiedlicher Konzentration herzustellen. Damit kann dem in der chemischen Industrie und verwandten Industriezweigen, z.B. in der Nahrungsmittelindustrie, Papierindustrie, und insbesondere in Getränkenbetrieben und Brauereien erforderlicher Bedarf an Peroxycarbonsäuren, insbesondere Peroxyessigsäure, als Desinfektionsmittel, entsprochen werden. In Getränkebetrieben, wie z.B. Mineralbrunnen und Brauereien, wird Peroxyessigsäure z.B. als Desinfektionsmittel zur Entkeimung von Spülzonen der Flaschenreinigungsanlagen eingesetzt; diese Anlagen erfordern eine andauernde und gleichbleibende Dosierung von ca. 10 bis 20 mg Peroxyessigsäure pro Liter zugeführtem Frischwasser. In Flaschenreinigungsanlagen von üblicher Größe werden pro Stunde etwa 10 bis 20 m³ Frischwasser verbraucht. Daraus berechnet sich ein maximaler Bedarf an 400 g Peroxyessigsäure/Stunde, der, wie das vorstehend gezeigt wurde, mit dem erfindungsgemäßen Verfahren und der erfindungsgemäßen Vorrichtung leicht und in regelbarer und auf für das Bedienungspersonal sichere Weise in einem geschlossenen Kreislauf und unter Vermeidung einer Zwischenlagerung bereitgestellt werden kann.

## Patentansprüche

1. Verfahren zur kontinuierlichen Herstellung von Peroxycarbonsäuren durch Umsetzung einer Carbonsäure mit Wasserstoffperoxid in einem wässerigen Medium in Gegenwart eines Katalysators,
**dadurch gekennzeichnet,**
daß man die Umsetzung bei einer Temperatur im Bereich von 40 bis 60°C durchführt.

2. Verfahren nach Anspruch 1,
**dadurch gekennzeichnet,**
daß man das Wasserstoffperoxid und die Carbonsäure einem Verweilreaktor zur Aufnahme des Reaktionsgemisches in 2 getrennten Zuleitungen kontinuierlich zuführt und die gebildete Peroxycarbonsäure nach Einstellung des Reaktionsgleichgewichts kontinuierlich vom Verweilreaktor abzieht.

3. Verfahren nach Anspruch 2,
**dadurch gekennzeichnet,**
daß die Konzentration des zugeführten Wasserstoffperoxids 30 bis 50 Gew.-% beträgt.

4. Verfahren nach einem der Ansprüche 1 bis 3,
**dadurch gekennzeichnet,**
daß man als Katalysator eine Mineralsäure verwendet.

5. Verfahren nach Anspruch 4,
**dadurch gekennzeichnet,**
daß man Schwefelsäure, Phosphorsäure oder Salpetersäure verwendet.

6. Verfahren nach Anspruch 5,
**dadurch gekennzeichnet,**
daß man Schwefelsäure verwendet.

7. Verfahren nach einem der vorhergehenden Ansprüche,
**dadurch gekennzeichnet,**
daß die Umsetzung bei einer Temperatur im Bereich von 50 bis 60°C durchgeführt wird.

8. Verfahren nach einem der vorhergehenden Ansprüche,
**dadurch gekennzeichnet,**
daß die Umsetzung bei einer Temperatur von 50°C durchgeführt wird.

9. Verfahren nach einem der vorhergehenden Ansprüche,
**dadurch gekennzeichnet,**
daß die Konzentration der Carbonsäure im Reaktionsmedium 10 bis 50 Gew.-%, insbesondere 30 bis 40 Gew.-% beträgt.

10. Verfahren nach einem der vorhergehenden Ansprüche,
**dadurch gekennzeichnet,**
daß die Konzentration der Peroxycarbonsäure im Reaktionsmedium 1 bis 30 Gew.-% beträgt.

11. Verfahren nach einem der vorhergehenden Ansprüche,
**dadurch gekennzeichnet,**
daß man als Carbonsäure eine C₁-C₆ -Monocarbonsäure oder Oligocarbonsäure einsetzt.

12. Verfahren nach Anspruch 11,
**dadurch gekennzeichnet,**
daß man Ameisensäure oder Essigsäure einsetzt.

13. Verfahren nach einem der vorhergehenden Ansprüche,
**dadurch gekennzeichnet,**
daß man die vom Verweilreaktor abgezogene Peroxycarbonsäure direkt dem Verwendungszweck, z.B. der zur behandelnden Leitung oder dem Desinfektionsmittelkreislauf, zuführt.

14. Vorrichtung zur Durchführung des Verfahrens nach einem der Ansprüche 1 bis 13, umfassend: einen Verweilreaktor (1) zur Aufnahme des Reaktionsmediums, der mit einer Heizung (10) versehen ist, zwei mit Rückschlagventilen (9,9) versehene Zuleitungen (2,2), die mit je einer Dosierpumpe (3,3) zur Zuführung der Reaktionskomponenten aus den Vorratsbehältern (11,12) in die Zuleitungen (2,2) versehen sind.

15. Vorrichtung nach Anspruch 14,
**dadurch gekennzeichnet,**
daß die Heizung (10) des Verweilreaktors (1) eine Innenheizung, z.B. in Form einer elektrischen Heizschlange, oder eine Außenheizung, z.B. in Form eines den Reaktor umgebenden Hohlmantels, ist.

16. Vorrichtung nach Anspruch 14 oder 15,
**dadurch gekennzeichnet,**
daß sie am Ausgang des Reaktors einen Drucksensor (4) aufweist.

17. Vorrichtung nach einem der Ansprüche 14 bis 16,
**dadurch gekennzeichnet,**
daß der Drucksensor (4) mit dem Magnetventil (17) gekoppelt ist, welches ermöglicht, daß bei Überschreitung eines Druckgrenzwertes die Vorrichtung automatisch mit Wasser gespült wird.

18. Vorrichtung nach einem der Ansprüche 14 bis 17,
**dadurch gekennzeichnet,**
daß sie mit einem mechanischenÜberdruckventil (20) für einen Druckabbau im Reaktor versehen ist.

19. Vorrichtung nach einem der Ansprüche 14 bis 18,
**dadurch gekennzeichnet,**
daß sie mit einem Endlüftungsventil (5) und einem Ablaßventil (6) zur Entlüftung oder Entleerung bzw. mit einem Magnetventil (17) zur Spülung mit Wasser versehen ist.

20. Vorrichtung nach Anspruch 19,
**dadurch gekennzeichnet,**
daß sie mit einem Neutralisationsgefäß (7) versehen ist, über das die Ableitung der durch die Wasserspülung ausgedrückten bzw. abgelassenen Peroxycarbonsäure erfolgt.

21. Vorrichtung nach einem der Ansprüche 14 bis 20,
**dadurch gekennzeichnet,**
daß sie mit geeigneten Einrichtungen zur direkten Dosierung der aus dem Verweilreaktor abgezogenen Peroxycarbonsäure zum Ort der Verwendung, z.B. zu den zu behandelnden Leitungen oder einem Desinfektionsmittelkreislauf, versehen ist.

22. Vorrichtung nach einem der Ansprüche 14 bis 21,
**dadurch gekennzeichnet,**
daß mindestens die mit den Ausgangs- oder Endprodukten in Berührung kommenden Teile der Vorrichtung aus einem gegenüber diesen Substanzen korrosionsbeständigen Materialien bestehen.

23. Vorrichtung nach Anspruch 22,
**dadurch gekennzeichnet,**
daß das Material Kunststoff ist.

24. Vorrichtung nach Anspruch 23,
**dadurch gekennzeichnet,**
daß der Kunststoff Polyvinylidenfluorid (PVDF) und/oder Polytetrafluorethylen (PTFE) ist.

25. Vorrichtung nach Anspruch 22,
**dadurch gekennzeichnet,**
daß das Material Glas ist.

26. Vorrichtung nach Anspruch 22,
**dadurch gekennzeichnet,**
daß das Material ein gegebenenfalls mit einem Kunststoff oder Glas beschichteter korrosionsbeständiger Stahl und/oder NE-Metall ist.

27. Vorrichtung nach Anspruch 22,
**dadurch gekennzeichnet,**
daß das Material ein gegebenenfalls durch andere geeignete Passivierungsmaßnahmen korrosionsbeständig gemachter Stahl oder NE-Metall ist.

28. Vorrichtung nach den Ansprüchen 14 bis 21,
**dadurch gekennzeichnet,**
daß die elektronische Steuerungs-, Regelungs- und Überwachungseinheit durch eine speicherprogrammierbare Steuerung, einen Einplatinen-Mikrocomputer bzw. ein Mikrocomputer-Entwicklungssystem, einen Personal Computer mit digitalen und analogen Ein- und Ausgabebaugruppen, sowie diskret aufgebaute elektronische oder elektromechanische Schaltungen sowie eine Kombination aus den Vorgenannten realisiert werden kann.
